# EUROPEAN PATENT APPLICATION

(11) **EP 4 752 136 A1**
(43) Date of publication of application: **03.06.2026**
(21) Application number: 24845297.1
(22) Date of filing: 26.06.2024
(51) Int. Cl.: C07D 233/90, C07F 3/02

(54) **METHOD FOR PRODUCING 2-ETHYLSULFONYL-1-HETEROARYLETHANONE COMPOUND**

(30) Priority: 24.07.2023 JP 2023119877
(71) Applicant: Nippon Soda Co., Ltd., Tokyo 100-7010 (JP)
(72) Inventor: OOKA Hirohito, Odawara-shi, Kanagawa 250-0280 (JP); IKEDA Yuto, Odawara-shi, Kanagawa 250-0280 (JP); KITAYAMA Takashi, Odawara-shi, Kanagawa 250-0280 (JP)
(74) Representative: Sonnenhauser, Thomas Martin
(86) International application number: PCT/JP2024/023147
(87) International publication number: WO 2025/022915

(57) **Abstract**

A method for producing a 2-(ethanesulfonyl)-1-heteroarylethan-1-one compound, the method including:
chemically reacting (methanesulfonyl)ethane with an alkylmagnesium halide to obtain an (ethanesulfonyl)methylmagnesium halide; and
chemically reacting the (ethanesulfonyl)methylmagnesium halide with a heteroarylcarboxylic acid ester compound, such as a substituted or unsubstituted 5- to 6-membered heteroaryl carboxylic acid alkyl ester or a substituted or unsubstituted fused bicyclic 9-membered heteroaryl carboxylic acid alkyl ester.

## Description

### TECHNICAL FIELD

The present invention relates to a novel method for producing a 2-ethylsulfonyl-1-heteroarylethanone compound.

Priority is claimed on Japanese Patent Application No. 2023-119877, filed July 24, 2023, the content of which is incorporated herein by reference.

### BACKGROUND ART

Heterocyclic building blocks are one of those that are important among groups of structural fragments used for the organic synthesis of compounds that serve as drug substances for medicines, agricultural chemicals, and the like. 2-(ethanesulfonyl)-1-heteroarylethan-1-one compounds are known as one type of heterocyclic building blocks.

For example, Patent Document 1 discloses the production of 1-{5-[(1Z)-2-chloro-3,3,3-trifluoropropan-1-en-1-yl]-1-methyl-1H-imidazol-2-yl}-2-(ethanesulfonyl)ethan-1-one (English name: 1-{5-[(1Z)-2-chloro-3,3,3-trifluoroprop-1-en-1-yl]-1-methyl-1H-imidazol-2-yl}-2-(ethanesulfonyl)ethan-1-one; Formula (A)) via the following process. In the Formula, Et represents an ethyl group.

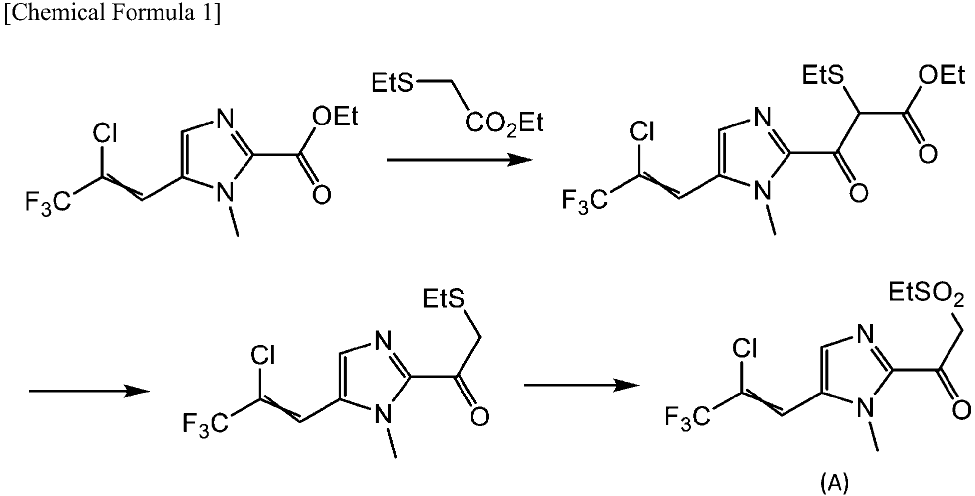

Patent Document 2 discloses that 1-(5-chloropyrazin-2-yl)-2-(ethanesulfonyl)ethan-1-one (1-(5-chloropyrazin-2-yl)-2-(ethanesulfonyl)ethan-1-one; Formula (B)) was obtained by: dropwise adding triethylamine to a mixture of magnesium chloride and tetrahydrofuran; dropwise adding a mixture of 1-(ethanesulfonyl)-2-propanone and tetrahydrofuran to the obtained mixture and stirring at 20°C; dropwise adding a mixture of 5-chloro-2-pyrazinecarboxylic acid chloride and toluene to the obtained mixture and stirring at 20°C; cooling the obtained mixture to 0°C; dropwise adding a mixture of 35% hydrochloric acid and water thereto, followed by adding a mixture of toluene, water, and tetrahydrofuran and stirring at 20°C; separating the resulting mixture; washing the obtained organic layer with an aqueous sodium bicarbonate solution; filtering this organic layer obtain a filtrate; evaporating the filtrate to dryness to obtain a dried product; and purifying this dried product by crystallization.

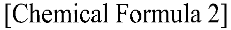

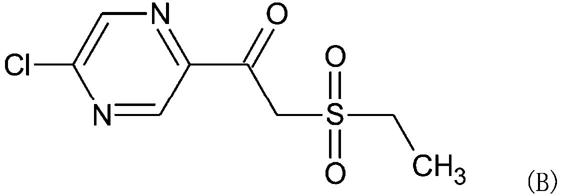

Patent Document 3 discloses that 2-(ethanesulfonyl)-1-(5-iodo-1-methyl-1H-imidazol-2-yl)ethan-1-one (English name: 2-(ethanesulfonyl)-1-(5-iodo-1-methyl-1H-imidazol-2-yl)ethan-1-one; Formula (C)) was obtained by: dissolving 2,5-diiodo-1-methyl-1H-imidazole in tetrahydrofuran; cooling the resulting solution to -70°C; dropwise adding an n-hexane solution of n-butyllithium thereto; stirring the resulting mixture at -70°C; adding 2-chloro-N-methoxy-N-methylacetamide to the obtained mixture; stirring the resulting mixture at -70°C; pouring the obtained liquid into a saturated aqueous solution of ammonium chloride; adding ethyl acetate; stirring and separating the resulting mixture; washing the obtained organic layer with saturated brine; drying the resulting product over anhydrous magnesium sulfate; filtering the resulting product to obtain a filtrate; concentrating the filtrate under reduced pressure; dissolving the obtained concentrate (containing 2-chloro-1-(5-iodo-1-methyl-1H-imidazol-2-yl)ethan-1-one) in a mixture of N,N-dimethylformamide and tetrahydrofuran; stirring the resulting solution at 0°C; adding sodium ethyl mercaptan thereto; stirring the resulting mixture at room temperature; pouring the obtained liquid into water; adding ethyl acetate; stirring and separating the resulting mixture; washing the obtained organic layer with saturated brine; drying the resulting product over anhydrous magnesium sulfate; filtering the resulting product to obtain a filtrate; concentrating the filtrate under reduced pressure; purifying the obtained concentrate to obtain 2-(ethylthio)-1-(5-iodo-1-methyl-1H-imidazol-2-yl)ethan-1-one, which was dissolved in dichloromethane; stirring the resulting solution at 0°C; adding meta-chloroperoxybenzoic acid thereto; stirring the resulting mixture; pouring the obtained liquid into a mixture of a saturated aqueous solution of sodium bicarbonate and a saturated aqueous solution of sodium thiosulfate; adding dichloromethane thereto; stirring and separating the resulting mixture; washing the obtained organic layer with saturated brine; drying the resulting product over anhydrous magnesium sulfate; filtering the resulting product; concentrating the obtained filtrate under reduced pressure; and purifying the obtained concentrate.

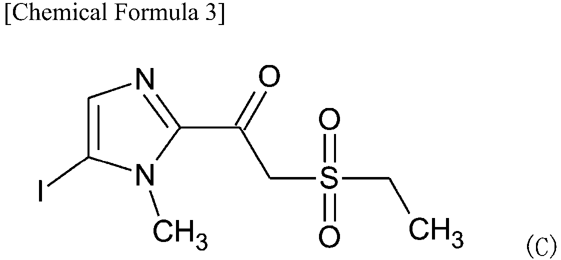

### Citation List

### Patent Documents

Patent Document 1: International Patent Publication No. 2023/090345
Patent Document 2: International Patent Publication No. 2018/194077
Patent Document 3: International Patent Publication No. 2020/071304

### SUMMARY OF INVENTION

### Technical Problem

An object of the present invention is to provide a novel method for producing a 2-(ethanesulfonyl)-1-heteroarylethan-1-one compound, which is one of the heterocyclic building blocks used for the organic synthesis of compounds that serve as drug substances for medicines, agricultural chemicals, and the like.

### Solution to Problem

As a result of intensive research in order to achieve the above object, the present invention including the following aspects has been completed.
[1] A method for producing a 2-(ethanesulfonyl)-1-heteroarylethan-1-one compound (for example, a compound represented by formula (5)), the method including chemically reacting an (ethanesulfonyl)methylmagnesium halide (a compound represented by formula (3)) with a heteroaryl carboxylic acid ester compound (for example, a compound represented by formula (4)), 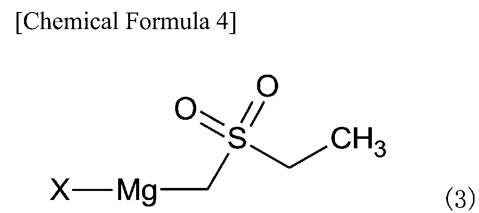 (In the formula (3), X is a halogeno group.) 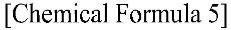 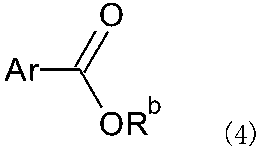 (In the formula (4), R^{b} is an alkyl group, and Ar is a substituted or unsubstituted heteroaryl group.) 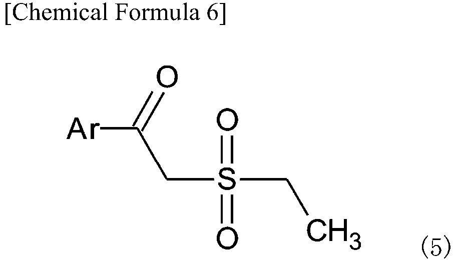 (In the formula (5), Ar is the same substituted or unsubstituted heteroaryl group as Ar in the formula (4).)

The method according to [1], further including chemically reacting (methanesulfonyl)ethane (a compound represented by formula (1)) with an alkylmagnesium halide (a compound represented by formula (2), sometimes also referred to as an alkyl Grignard reagent) to obtain the aforementioned (ethanesulfonyl)methylmagnesium halide (a compound represented by formula (3)), 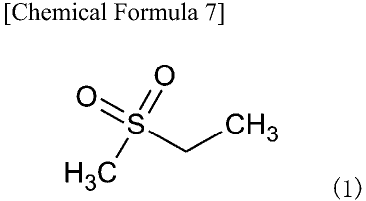 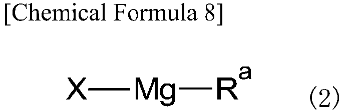 (In the formula (2), X is the same halogeno group as X in the formula (3), and R^{a} is an alkyl group.)

[3] The method according to [1] or [2], wherein the aforementioned heteroaryl carboxylic acid ester compound is a substituted or unsubstituted 5- to 6-membered heteroaryl carboxylic acid alkyl ester or a substituted or unsubstituted fused bicyclic 9-membered heteroaryl carboxylic acid alkyl ester.

[4] A method for producing (ethanesulfonyl)methylmagnesium halide (a compound represented by formula (3)), the method including chemically reacting (methanesulfonyl)ethane (a compound represented by formula (1)) with an alkylmagnesium halide (a compound represented by formula (2), sometimes also referred to as an alkyl Grignard reagent).

### Advantageous Effects of Invention

According to the production method of the present invention, it is possible to obtain an (ethanesulfonyl)methylmagnesium halide (a compound represented by formula (3)) and a 2-(ethanesulfonyl)-1-heteroarylethan-1-one compound (for example, a compound represented by formula (5)) at high yields. A 2-(ethanesulfonyl)-1-heteroarylethan-1-one compound is useful as a heterocyclic building block for producing the heteroarylazole compound and pest control agent described in Patent Document 3, and the like.

### DESCRIPTION OF EMBODIMENTS

One embodiment of the production method of the present invention is a method for producing a 2-(ethanesulfonyl)-1-heteroarylethan-1-one compound, which includes chemically reacting an (ethanesulfonyl)methylmagnesium halide with a heteroaryl carboxylic acid ester compound.

Another embodiment of the production method of the present invention is a method for producing a 2-(ethanesulfonyl)-1-heteroarylethan-1-one compound, which includes chemically reacting (methanesulfonyl)ethane with an alkylmagnesium halide to obtain an (ethanesulfonyl)methylmagnesium halide, and chemically reacting the aforementioned (ethanesulfonyl)methylmagnesium halide with a heteroarylcarboxylic acid ester compound.

Another embodiment of the production method of the present invention is a method for producing an (ethanesulfonyl)methylmagnesium halide, which includes chemically reacting (methanesulfonyl)ethane with an alkylmagnesium halide.

In the present invention, the term "unsubstituted" means that it is composed only of a group which becomes a mother nucleus. When it is described only by the name of the group which becomes the mother nucleus without being described as "substituted", it means "unsubstituted" unless otherwise stated.

On the other hand, the term "substituted" means that any hydrogen atom of a group which becomes a mother nucleus is substituted with a group (substituent) having the same or different structure as that of the mother nucleus. Therefore, a "substituent" is another group bonded to a group which becomes a mother nucleus. The number of substituents may be one, or two or more. The two or more substituents may be the same or different.

The terms "C₁₋₆" and the like mean that the number of carbon atoms in the group which becomes a mother nucleus is 1 to 6, and so on. The number of carbon atoms does not include the number of carbon atoms present in the substituent. For example, a butyl group having an ethoxy group as a substituent is classified as a C₂ alkoxy C₄ alkyl group.

A "substituent" is not particularly limited as long as it is chemically acceptable and has the effects of the present invention.

Hereinafter, groups which can be a "substituent" are exemplified.
A C₁₋₆ alkyl group such as a methyl group, an ethyl group, an n-propyl group, an i-propyl group, an n-butyl group, an s-butyl group, an i-butyl group, a t-butyl group, an n-pentyl group, and an n-hexyl group;
a C₂₋₆ alkenyl group such as a vinyl group, a 1-propenyl group, a 2-propenyl group (allyl group), a 1-butenyl group, a 2-butenyl group, a 3-butenyl group, a 1-methyl-2-propenyl group, and a 2-methyl-2-propenyl group;
a C₂₋₆ alkynyl group such as an ethynyl group, a 1-propynyl group, a 2-propynyl group, a 1-butynyl group, a 2-butynyl group, a 3-butynyl group, and a 1-methyl-2-propynyl group;
a C₃₋₆ cycloalkyl group such as a cyclopropyl group, a cyclobutyl group, a cyclopentyl group and a cyclohexyl group;
a phenyl group;
a 3- to 6-membered heterocyclyl group;
a C₁₋₆ alkoxy group such as a methoxy group, an ethoxy group, an n-propoxy group, an i-propoxy group, an n-butoxy group, an s-butoxy group, an i-butoxy group, and a t-butoxy group;
a C₆₋₁₀ aryloxy group such as a phenoxy group and a naphthoxy group;
a 5- to 6-membered heteroaryloxy group such as a thiazolyloxy group and a pyridyloxy group;
a C₁₋₆ alkoxycarbonyl group such as a methoxycarbonyl group, an ethoxycarbonyl group, an n-propoxycarbonyl group, an i-propoxycarbonyl group, an n-butoxycarbonyl group and a t-butoxycarbonyl group;
a halogeno group such as a fluoro group, a chloro group, a bromo group, and an iodo group;
a C₁₋₆ haloalkyl group such as a chloromethyl group, a chloroethyl group, a trifluoromethyl group, a 1,2-dichloro-n-propyl group, a 1-fluoro-n-butyl group and a perfluoro-n-pentyl group;
a C₁₋₆ haloalkoxy group such as a trifluoromethoxy group, a 2-chloro-n-propoxy group and a 2,3-dichlorobutoxy group;
a formylamino group;
a C₁₋₆ alkylcarbonylamino group such as an acetylamino group, a propanoylamino group, a butyrylamino group and an i-propylcarbonylamino group;
a C₁₋₆ alkoxycarbonylamino group such as a methoxycarbonylamino group, an ethoxycarbonylamino group, an n-propoxycarbonylamino group and an i-propoxycarbonylamino group;
an unsubstituted or substituted aminocarbonyl group such as an aminocarbonyl group, a dimethylaminocarbonyl group, a phenylaminocarbonyl group and an N-phenyl-N-methylaminocarbonyl group;
a C₁₋₆ alkylthio group such as a methylthio group, an ethylthio group, an n-propylthio group, an i-propylthio group, an n-butylthio group, an i-butylthio group, an s-butylthio group and a t-butylthio group;
a C₁₋₆ haloalkylthio group such as a trifluoromethylthio group and a 2,2,2-trifluoroethylthio group;
a C₁₋₆ alkylsulfonyl group such as a methylsulfonyl group, an ethylsulfonyl group and a t-butylsulfonyl group;
a C₁₋₆ haloalkylsulfonyl group such as a trifluoromethylsulfonyl group and a 2,2,2-trifluoroethylsulfonyl group;
a cyano group; and a nitro group.

Further, in these "substituents", any hydrogen atom in the substituent may be substituted with a group having a different structure. Examples of the "substituent" in this case include a C₁₋₆ alkyl group, a C₁₋₆ haloalkyl group, a C₁₋₆ alkoxy group, a C₁₋₆ haloalkoxy group, a halogeno group, a cyano group and a nitro group.

Further, the above-described "3- to 6-membered heterocyclyl group" includes 1 to 4 hetero atoms selected from the group consisting of a nitrogen atom, an oxygen atom and a sulfur atom as constituent atoms of the ring. The heterocyclyl group may be either monocyclic or polycyclic. As long as the polycyclic heterocyclyl group includes at least one heterocyclic ring, the remaining ring may be any of a saturated alicyclic ring, an unsaturated alicyclic ring or an aromatic ring. Examples of the "3- to 6-membered heterocyclyl group" include a 3- to 6-membered saturated heterocyclyl group, a 5- to 6-membered heteroaryl group and a 5- to 6-membered partially unsaturated heterocyclyl group.

Examples of the 3- to 6-membered saturated heterocyclyl group include an aziridinyl group, an epoxy group, a pyrrolidinyl group, a tetrahydrofuranyl group, a thiazolidinyl group, a piperidyl group, a piperazinyl group, a morpholinyl group, a dioxolanyl group, and a dioxanyl group.

Examples of the 5-membered heteroaryl group include a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group and a tetrazolyl group.

Examples of the 6-membered heteroaryl group include a pyridyl group, a pyrazinyl group, a pyrimidinyl group, a pyridazinyl group and a triazinyl group.

Examples of the 5-membered partially unsaturated heterocyclyl group include a pyrrolinyl group, a dihydrofuranyl group, an imidazolinyl group, a pyrazolinyl group, an oxazolinyl group, and an isoxazolinyl group.

Examples of the 6-membered partially unsaturated heterocyclyl group include a dihydropyranyl group.

The 2-(ethanesulfonyl)-1-heteroarylethan-1-one compound obtained by one embodiment of the production method of the present invention is one of heterocyclic building blocks. The 2-(ethanesulfonyl)-1-heteroarylethan-1-one compound can be used for the organic synthesis of compounds that serve as drug substances for medicines, agricultural chemicals, and the like.

The 2-(ethanesulfonyl)-1-heteroarylethan-1-one compounds include 2-(ethanesulfonyl)-1-unsubstituted heteroarylethan-1-ones and 2-(ethanesulfonyl)-1-substituted heteroarylethan-1-ones (see formula (5)).

Examples of the heteroaryl in the 2-(ethanesulfonyl)-1-heteroarylethan-1-one compound, or in the 2-(ethanesulfonyl)-1-unsubstituted heteroarylethan-1-one and 2-(ethanesulfonyl)-1-substituted heteroarylethan-1-one include a substituted or unsubstituted 5- to 6-membered heteroaryl or a substituted or unsubstituted fused bicyclic 9-membered heteroaryl, and preferred examples thereof include a substituted or unsubstituted 5- to 6-membered nitrogen atom-containing heteroaryl or a substituted or unsubstituted nitrogen atom-containing fused bicyclic 9-membered heteroaryl. 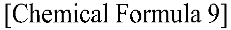 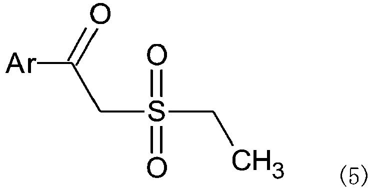 (In the formula (5), Ar is the same unsubstituted or substituted heteroaryl group as Ar in the formula (4).)

The heteroaryl group represented by Ar is an aromatic ring containing a chemically acceptable number of hetero atoms being at least one selected from the group consisting of a nitrogen atom, an oxygen atom and a sulfur atom as constituent atoms of the ring.

A fused bicyclic 9-membered heteroaryl group is formed by fusing one aromatic ring containing a chemically acceptable number of hetero atoms being at least one selected from the group consisting of a nitrogen atom, an oxygen atom and a sulfur atom as constituent atoms of the ring, to another ring. Another ring may be any of a saturated alicyclic ring, an unsaturated alicyclic ring, an aromatic alicyclic ring, a saturated heterocyclic ring, an unsaturated heterocyclic ring, and an aromatic heterocyclic ring.

Examples of the 5-membered heteroaryl group include a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group and a tetrazolyl group. Of these, an imidazolyl group is preferred.

Examples of the 6-membered heteroaryl group include a pyridyl group, a pyrazinyl group, a pyrimidinyl group, a pyridazinyl group and a triazinyl group.

Examples of the fused bicyclic 9-membered heteroaryl group include an indolyl group, an isoindolyl group, a benzofuranyl group, a benzothienyl group, an indazolyl group, a benzimidazolyl group, a benzoxazolyl group, a benzisoxazolyl group, a benzothiazolyl group, a benzisothiazolyl group, an imidazopyridyl group, and an imidazopyridazinyl group.

As a substituent on the "5- or 6-membered heteroaryl group", a halogeno group, a substituted or unsubstituted C₁₋₆ alkyl group, a substituted or unsubstituted C₂₋₆ alkenyl group, a substituted or unsubstituted C₂₋₆ alkynyl group, a hydroxyl group, a substituted or unsubstituted C₁₋₆ alkoxy group, a substituted or unsubstituted C₃₋₆ cycloalkyl group, a substituted or unsubstituted C₁₋₆ alkylthio group, a substituted or unsubstituted C₁₋₆ alkylsulfonyl group, or a cyano group is preferred, and a substituted or unsubstituted C₁₋₆ alkyl group or a substituted or unsubstituted alkenyl group is more preferred.

As a substituent on the "C₁₋₆ alkyl group," "C₂₋₆ alkenyl group," "C₂₋₆ alkynyl group," "C₁₋₆ alkoxy group," "C₁₋₆ alkylthio group," and "C₁₋₆ alkylsulfonyl group", a halogeno group, a C₁₋₆ alkoxy group, a C₁₋₆ haloalkoxy group, or a cyano group is preferred.

As a substituent on the "C₃₋₆ cycloalkyl group", a halogeno group, a C₁₋₆ alkyl group, a C₁₋₆ haloalkyl group, a C₁₋₆ alkoxy group, a C₁₋₆ haloalkoxy group, or a cyano group is preferred.

The production method of the present embodiment is preferably suitable for obtaining a compound represented by formula (5a), more preferably a compound represented by formula (5b) or formula (5c). As described in Patent Document 1, the compound represented by the formula (5a), formula (5b), or formula (5c) is useful as a heterocyclic building block for producing the heteroarylazole compound and pest control agent described in Patent Document 3, and the like. The wavy line in the formula (5b) represents a single bond of undetermined three-dimensional structure (undefined stereo bond) or a mixture of multiple three-dimensional structures resulting from the above single bond.

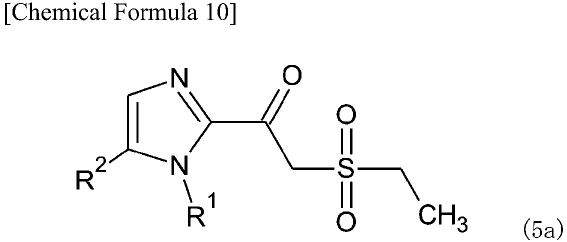

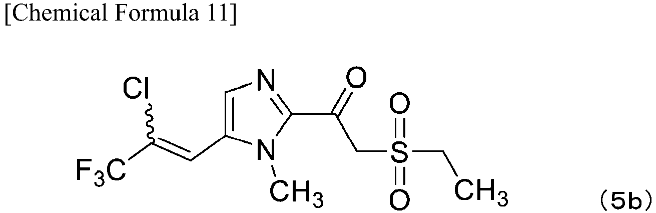

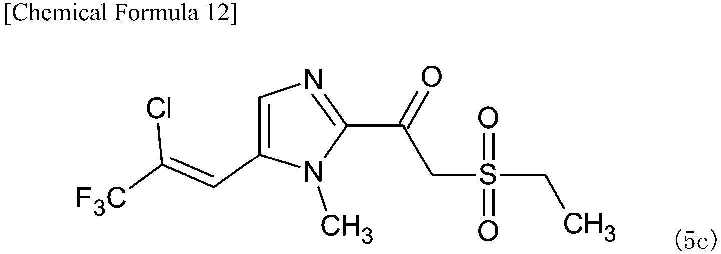

In the formula (5a), R¹ is a C₁₋₆ alkyl group.

The C₁₋₆ alkyl group represented by R¹ may be linear or branched. Examples of the C₁₋₆ alkyl group represented by R¹ include a methyl group, an ethyl group, an n-propyl group, an n-butyl group, an n-pentyl group, an n-hexyl group, an i-propyl group, an i-butyl group, an s-butyl group, a t-butyl group, an i-pentyl group, a neopentyl group, a 2-methylbutyl group, a 2,2-dimethylpropyl group and an i-hexyl group. Of these, a methyl group is preferred.

In the formula (5a), R² is a substituted or unsubstituted C₁₋₆ alkyl group or a substituted or unsubstituted C₂₋₆ alkenyl group.

The "C₁₋₆ alkyl group" represented by R² can be the same as that represented by R¹.

Examples of the "C₂₋₆ alkenyl group" represented by R² include a vinyl group, a 1-propenyl group, a 2-propenyl group, a 1-butenyl group, a 2-butenyl group, a 3-butenyl group, a 1-methyl-2-propenyl group, a 2-methyl-2-propenyl group, a 1-pentenyl group, a 2-pentenyl group, a 3-pentenyl group, a 4-pentenyl group, a 1-methyl-2-butenyl group, a 2-methyl-2-butenyl group, a 1-hexenyl group, a 2-hexenyl group, a 3-hexenyl group, a 4-hexenyl group and a 5-hexenyl group. Of these, a vinyl group is preferred.

As a substituent on the "C₁₋₆ alkyl group" and "C₂₋₆ alkenyl group" represented by R², a halogeno group, a C₁₋₆ alkoxy group, a C₁₋₆ haloalkyl group, a C₁₋₆ haloalkoxy group, or a cyano group is preferred, and a halogeno group or a C₁₋₆ haloalkyl group is more preferred.

Examples of the halogeno-substituted C₁₋₆ alkyl group (sometimes referred to as a C₁₋₆ haloalkyl group) represented by R² include a fluoromethyl group, a difluoromethyl group, a trifluoromethyl group, a 2,2,2-trifluoroethyl group, a pentafluoroethyl group, a 3,3,3-trifluoropropyl group, a 2,2,3,3,3-pentafluoropropyl group, a 1-chloro-2,2,3,3,3-pentafluoropropyl group, a 1,2,2,3,3,3-hexafluoropropyl group, a perfluoropropyl group, a 2,2,2-trifluoro-1-trifluoromethylethyl group, a perfluoroisopropyl group, a 4-fluorobutyl group, a 1,4,4,4-tetrafluorobutyl group, a 2,2,3,3,4,4,4-heptafluorobutyl group, a 1,2,2,3,3,4,4,4-octafluorobutyl group, a perfluorobutyl group, a 3,3,3-trifluoro-2-trifluoromethylpropyl group, a 1-chloro-2,3,3,3-tetrafluoro-2-trifluoromethylpropyl group, a 1,2,3,3,3-pentafluoro-2-trifluoromethylpropyl group, a perfluoropentyl group, and a perfluorohexyl group.

Examples of the halogeno-substituted C₂₋₆ alkenyl group (sometimes referred to as a C₂₋₆ haloalkenyl group) represented by R² include a 2,3,3,3-tetrafluoro-1-propenyl group, a 3,3,3-trifluoro-1-propenyl group, a 2-chloro-3,3,3-trifluoro-1-propenyl group, a 2-bromo-3,3,3-trifluoro-1-propenyl group, a 3,3,3-trifluoro-2-trifluoromethyl-1-propenyl group, a 3,3,4,4,4-pentafluoro-1-butenyl group, a 2,3,3,4,4,4-hexafluoro-1-butenyl group, and a 2-chloro-3,3,4,4,4-pentafluoro-1-butenyl group. Of these, a 2-chloro-3,3,3-trifluoro-1-propenyl group is preferred. The 2-chloro-3,3,3-trifluoro-1-propenyl group may be composed of a mixture of E/Z stereoisomers, or may be composed only of a Z isomer or E isomer.

The (ethanesulfonyl)methylmagnesium halide used in the present embodiment is a compound represented by formula (3). The (ethanesulfonyl)methylmagnesium halide is a type of Grignard reagent. Since this Grignard reagent decomposes when acidic protons such as water are present, an (ethanesulfonyl)methylmagnesium halide is typically contained in an organic solvent used when preparing the (ethanesulfonyl)methylmagnesium halide.

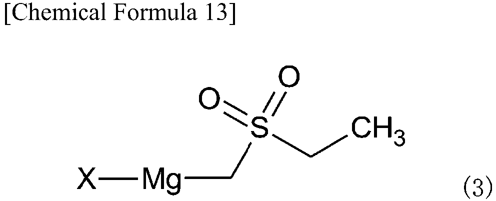

In the formula (3), X is a halogeno group. Examples of the halogeno group include a fluoro group, a chloro group, a bromo group, and an iodo group. Of these, a chloro group is preferred.

(ethanesulfonyl)methylmagnesium halides can be obtained in accordance with a general method for preparing a Grignard reagent. Examples of the general method for preparing a Grignard reagent include a chemical reaction of an alkyl halide with magnesium, a chemical reaction of causing another Grignard reagent to act on a highly acidic hydrocarbon, a metal-halogen exchange reaction between an alkyl halide and another Grignard reagent, and a transmetalation reaction between another organometallic compound and a magnesium halide.

The (ethanesulfonyl)methylmagnesium halide is preferably prepared in an organic solvent. Examples of the organic solvent include ethers such as diethyl ether, diisopropyl ether, diethylene glycol dimethyl ether, and tetrahydrofuran; halogenated hydrocarbons such as dichloromethane, chloroform, and 1,2-dichloroethane; aliphatic hydrocarbons such as pentane, hexane, cyclohexane, heptane, and octane; and aromatic hydrocarbons such as toluene and xylene. Of these, ethers such as diethyl ether and tetrahydrofuran, and halogenated hydrocarbons such as chloroform are preferred.

The (ethanesulfonyl)methylmagnesium halide can be obtained, for example, by the (ethanesulfonyl)methylmagnesium halide production method according to the present embodiment, that is, a method involving chemically reacting (methanesulfonyl)ethane with an alkyl Grignard reagent (alkylmagnesium halide).

The (methanesulfonyl)ethane (also known as ethyl methyl sulfone or methylsulfonylethane) used in the present embodiment is a compound represented by the formula (1) [CAS RN: 594-43-4]. A commercially available product of (methanesulfonyl)ethane may also be used.

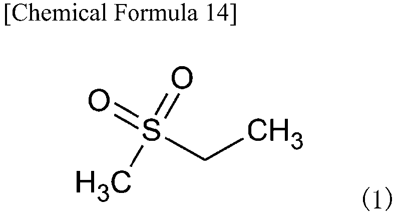

The alkylmagnesium halide used in the present embodiment is a compound represented by the formula (2). Alkylmagnesium halides are also known as alkyl Grignard reagents. Since alkyl Grignard reagents decompose when acidic protons such as water are present, alkylmagnesium halides are typically contained in an organic solvent used when preparing(ethanesulfonyl)methylmagnesium halides.

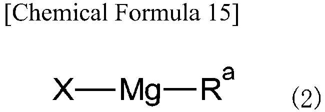

In the formula (2), X is the same halogeno group as X in the formula (3). In the formula (2), R^{a} is an alkyl group. Since R^{a} is eliminated due to the chemical reaction with (methanesulfonyl)ethane and removed from the system, R^{a} is not particularly limited as long as it does not inhibit the chemical reaction, but an alkyl group composed of 1 to 6 carbon atoms is preferred. The alkyl group represented by R^{a} may be linear or branched. Examples of the alkyl group represented by R^{a} include a methyl group, an ethyl group, an n-propyl group, an n-butyl group, an n-pentyl group, an n-hexyl group, an i-propyl group, an i-butyl group, an s-butyl group, a t-butyl group, an i-pentyl group, a neopentyl group, a 2-methylbutyl group, a 2,2-dimethylpropyl group and an i-hexyl group. Of these, a methyl group and a t-butyl group are preferred, and a methyl group is more preferred. Alkylmagnesium halides can be obtained in accordance with a general method for preparing a Grignard reagent. For example, they can be obtained by a method involving chemically reacting an alkyl halide with magnesium. Alternatively, a commercially available product of alkylmagnesium halide may also be used.

Specific examples of alkylmagnesium halides include methyl magnesium chloride, ethyl magnesium chloride, t-butyl magnesium chloride, methyl magnesium bromide, ethyl magnesium bromide, t-butyl magnesium bromide, methyl magnesium iodide, ethyl magnesium iodide, and t-butyl magnesium iodide.

The amount of the alkylmagnesium halide used in a chemical reaction between (methanesulfonyl)ethane and an alkylmagnesium halide (hereinafter sometimes referred to as chemical reaction I) is preferably from 1.0 to 2.0 moles, and more preferably from 1.0 to 1.2 moles, with respect to 1 mole of (methanesulfonyl)ethane. The amount of the organic solvent used in the chemical reaction between (methanesulfonyl)ethane and an alkylmagnesium halide is preferably from 5 to 500 parts by mass, with respect to 1 part by mass of (methanesulfonyl)ethane.

During the chemical reaction I, the temperature is preferably set to 0°C or higher and equal to or lower than the boiling point of the organic solvent used, and the pressure is preferably set to 0.1 to 1 MPa, more preferably from 0.1 to 0.5 MPa, and still more preferably 0.1 MPa. The time required for the chemical reaction I (average residence time in a reactor) is not particularly limited, but is, for example, from 0.5 to 24 hours. The chemical reaction I is preferably carried out under an inert gas atmosphere.

The chemical reaction I is carried out with no limitation by the procedure therefor, the reaction apparatus used therefor, and the like. For example, an organic solvent and (methanesulfonyl)ethane are mixed and stirred to obtain mixture A, and mixture B of an alkylmagnesium halide and an organic solvent is added dropwise or slowly to the mixture A while stirring. The organic solvent used in the mixture A and the organic solvent used in the mixture B may be the same or different. The reaction apparatus is not particularly limited, and a vessel-type batch reactor, a vessel-type flow reactor, a tubular reactor, and the like well known in the field of chemical engineering can be used. After completion of the chemical reaction I, the product may be purified, but it is preferable not to purify.

The heteroaryl carboxylic acid ester compound used in the present embodiment includes an unsubstituted or substituted heteroaryl carboxylic acid ester (see formula (4d)).

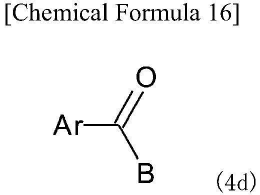

Ar in the formula (4d) is an unsubstituted heteroaryl group or a substituted heteroaryl group.

Since B in the formula (4d) is eliminated upon chemical reaction with an (ethanesulfonyl)methylmagnesium halide and ultimately removed from the system, there are no particular restrictions thereon as long as it does not inhibit the chemical reaction.

A preferred heteroaryl carboxylic acid ester compound used in the present embodiment includes an unsubstituted or substituted heteroaryl carboxylic acid alkyl ester (see formula (4)).

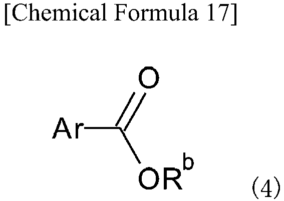

In the formula (4), R^{b} is an alkyl group, and Ar is an unsubstituted or substituted heteroaryl group. Ar in the formula (4) is as described for Ar in the formula (5).

Since OR^{b} in the formula (4) is eliminated upon chemical reaction with an (ethanesulfonyl)methylmagnesium halide and ultimately removed from the system, R^{b} is not particularly limited as long as it does not inhibit the chemical reaction, but an alkyl group composed of 1 to 6 carbon atoms is preferred. The alkyl group represented by R^{b} may be linear or branched. Examples of the alkyl group represented by R^{b} include a methyl group, an ethyl group, an n-propyl group, an n-butyl group, an n-pentyl group, an n-hexyl group, an i-propyl group, an i-butyl group, an s-butyl group, a t-butyl group, an i-pentyl group, a neopentyl group, a 2-methylbutyl group, a 2,2-dimethylpropyl group and an i-hexyl group. Of these, a methyl group or an ethyl group is preferred, and an ethyl group is more preferred.

In the present embodiment, a heteroaryl carboxylic acid ester compound corresponding to the compound represented by formula (5a), formula (5b), or formula (5c) can be used (formula (4a), formula (4b), or formula (4c)). The wavy line in the formula (4b) represents a single bond of undetermined three-dimensional structure (undefined stereo bond) or a mixture of multiple three-dimensional structures resulting from the above single bond. R^{b} in the formula (4b) or formula (4c) is preferably an ethyl group.

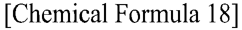

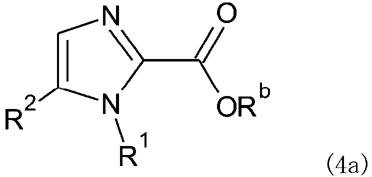

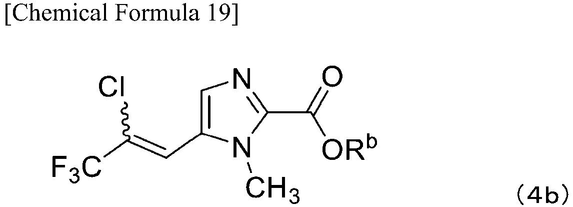

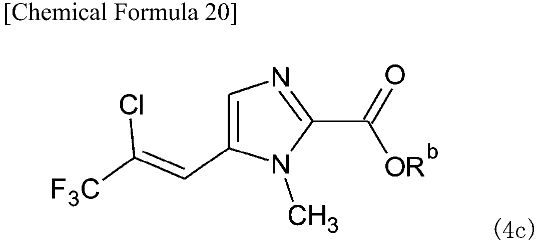

An organic solvent can be used in a chemical reaction between an (ethanesulfonyl)methylmagnesium halide and a heteroaryl carboxylic acid ester compound (hereinafter sometimes referred to as chemical reaction II). Examples of the organic solvent that can be used in the chemical reaction II include ethers such as diethyl ether, diisopropyl ether, diethylene glycol dimethyl ether, and tetrahydrofuran; halogenated hydrocarbons such as dichloromethane, chloroform, and 1,2-dichloroethane; aliphatic hydrocarbons such as pentane, hexane, cyclohexane, heptane, and octane; and aromatic hydrocarbons such as toluene and xylene. Of these, ethers such as diethyl ether and tetrahydrofuran, or halogenated hydrocarbons such as chloroform are preferred.

The amount of the (ethanesulfonyl)methylmagnesium halide used in the chemical reaction II is preferably from 1.2 to 2.0 moles, and more preferably from 1.4 to 1.8 moles, with respect to 1 mole of the heteroaryl carboxylic acid ester compound. The amount of the organic solvent used in the chemical reaction II is preferably from 5 to 500 parts by mass, with respect to 1 part by mass of the heteroaryl carboxylic acid ester compound.

During the chemical reaction II, the temperature is preferably set from 0°C to 40°C, more preferably from 5°C to 35°C, and the pressure is preferably set to 0.1 to 1 MPa, more preferably from 0.1 to 0.5 MPa, and still more preferably 0.1 MPa. The time required for the chemical reaction II (average residence time in a reactor) is not particularly limited, but is, for example, from 0.5 to 24 hours. The chemical reaction II is preferably carried out under an inert gas atmosphere.

The chemical reaction II is carried out with no limitation by the procedure therefor, the reaction apparatus used therefor, and the like. For example, an organic solvent and a heteroaryl carboxylic acid ester compound are mixed and stirred to obtain mixture C, and mixture D of an (ethanesulfonyl)methylmagnesium halide and an organic solvent is added dropwise or slowly to the mixture C while stirring. The organic solvent used in the mixture C and the organic solvent used in the mixture D may be the same or different. The reaction apparatus is not particularly limited, and a vessel-type batch reactor, a vessel-type flow reactor, a tubular reactor, and the like well known in the field of chemical engineering can be used. After completion of the chemical reaction II, the product may or may not be purified. Purification can be carried out by a known technique, such as extraction, crystallization, and chromatography.

### Examples

The present invention will be described in more detail below with reference to Examples. The present invention is not limited by the following Examples.

### [Example 1] Production of 1-[5-(2-chloro-3,3,3-trifluoroprop-1-en-1-yl)-1-methyl-1H-imidazol-2-yl]-2-(ethylsulfonyl)-ethan-1-one

### [Step 1] Chemical reaction of (methanesulfonyl)ethane with methylmagnesium chloride

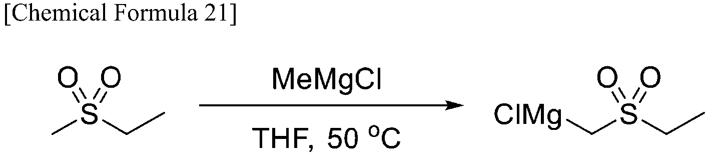

(Methanesulfonyl)ethane (837.8 mg, 7.7 mmol) was charged into reactor I, and the pressure reduction and replacement with nitrogen in the reactor I were repeated three times. Dehydrated tetrahydrofuran (5.0 mL) was added thereto and mixed to obtain mixture I. While stirring the mixture I at room temperature, a tetrahydrofuran solution of methylmagnesium chloride with a concentration of 2.81 M (2.75 mL, 7.7 mmol) was added dropwise to the reactor I over 3 minutes to obtain mixture II. After completion of the dropwise addition, the reactor I was heated to 50°C and the mixture II was stirred at 50°C for 4 hours. The reactor I was then returned to room temperature to obtain a tetrahydrofuran solution of (ethanesulfonyl)methylmagnesium chloride.

### [Step 2] Chemical reaction of (ethanesulfonyl)methylmagnesium chloride with ethyl 5-(2-chloro-3,3,3-trifluoroprop-1-en-1-yl)-1-methyl-1H-imidazole-2-carboxylate

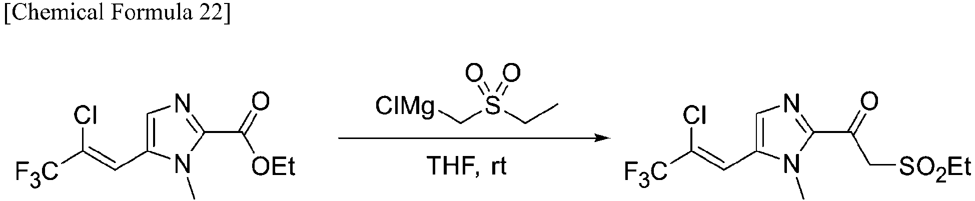

Ethyl 5-(2-chloro-3,3,3-trifluoroprop-1-en-1-yl)-1-methyl-1H-imidazole-2-carboxylate (1.37 g, 4.8 mmol) was charged into reactor II, and the pressure reduction and replacement with nitrogen in the reactor II were repeated three times. Dehydrated tetrahydrofuran (7.3 mL) was added thereto and mixed to obtain mixture III. While stirring the mixture III at room temperature, the tetrahydrofuran solution of (ethanesulfonyl)methylmagnesium chloride stored in the reactor I was transferred and added dropwise to the reactor II. After completion of the dropwise addition, the resulting mixture was stirred at room temperature for 2 hours. The reactor II was cooled to 0°C, and 2N hydrochloric acid was added thereto to terminate the chemical reaction, followed by extraction with ethyl acetate. The organic layer contained 97.6% of the target compound in terms of relative area ratio based on HPLC analysis. The organic layer was concentrated and purified by silica gel chromatography to obtain the target compound (1.55 g, yield: 93.4%).

¹H-NMR (400 MHz, CDCl₃) δ 1.47 (t, 3H), 3.29 (q, 2H), 4.05 (s, 3H), 4.86 (s, 2H), 7.14 (s, 1H), 8.06 (s, 1H).

As shown in the above Example, a 2-(ethanesulfonyl)-1-heteroarylethan-1-one compound can be obtained at a high yield by the method for producing a 2-(ethanesulfonyl)-1-heteroarylethan-1-one compound according to the present invention using (methanesulfonyl)ethane as a starting material.

### [Example 2] Chemical reaction of (methanesulfonyl)ethane with methylmagnesium chloride

(Methanesulfonyl)ethane (1.06 g, 9.8 mmol) was charged into reactor I, and the pressure reduction and replacement with nitrogen in the reactor I were repeated three times. Dehydrated tetrahydrofuran (6.3 mL) was added thereto and mixed to obtain mixture I. While stirring the mixture I at room temperature, a tetrahydrofuran solution of methylmagnesium chloride with a concentration of 2.81 M (3.5 mL, 9.8 mmol) was added dropwise to the reactor I over 3 minutes to obtain mixture II. After completion of the dropwise addition, the reactor I was heated to 50°C and the mixture II was stirred at 50°C for 2 hours. A small amount of liquid was withdrawn from the reactor I as sample a, and deuterated methanol was added thereto to terminate the chemical reaction. The resulting mixture was continued to be stirred in the reactor I at 50°C for 2 hours. A small amount of liquid was withdrawn from the reactor I as sample b, and deuterated methanol was added thereto to terminate the chemical reaction.

From the integrated values of the peaks derived from the methyl groups in (methanesulfonyl)ethane by ¹H-NMR for samples a and b, the deuteration percentages were 97.8% D after 2 hours and 100% D after 4 hours. In other words, 97.8% of (methanesulfonyl)ethane was converted to (ethanesulfonyl)methylmagnesium chloride after 2 hours, and 100% after 4 hours.

### [Example 3] Chemical reaction of (methanesulfonyl)ethane with t-butylmagnesium chloride

(Methanesulfonyl)ethane (514.0 mg, 4.7 mmol) was charged into reactor I, and the pressure reduction and replacement with nitrogen in the reactor I were repeated three times. After that, the reactor I was heated to 50°C, and while stirring (methanesulfonyl)ethane at 50°C, a tetrahydrofuran solution of t-butylmagnesium chloride with a concentration of 1.0 M (4.7 mL, 4.7 mmol) was added dropwise to the reactor I over 3 minutes to obtain mixture II. After completion of the dropwise addition, the mixture II was stirred at 50°C for 2 hours. A small amount of liquid was withdrawn from the reactor I as sample a, and deuterated methanol was added thereto to terminate the chemical reaction. The resulting mixture was continued to be stirred in the reactor I at 50°C for 2 hours. A small amount of liquid was withdrawn from the reactor I as sample b, and deuterated methanol was added thereto to terminate the chemical reaction.

From the integrated values of the peaks derived from the methyl groups in (methanesulfonyl)ethane by ¹H-NMR for samples a and b, the deuteration percentages were 80.3% D after 2 hours and 82.1% D after 4 hours. In other words, 80.3% of (methanesulfonyl)ethane was converted to (ethanesulfonyl)methylmagnesium chloride after 2 hours, and 82.1% after 4 hours.

### [Example 4]

Samples a and b were obtained in the same manner as in Example 2, with the exception that the temperature in the reactor I after the completion of the dropwise addition was changed from 50°C to 22°C. From the integrated values of the peaks derived from the methyl groups in (methanesulfonyl)ethane by ¹H-NMR for samples a and b, the deuteration percentages were 74.5% D after 2 hours and 89.7% D after 4 hours. In other words, 74.5% of (methanesulfonyl)ethane was converted to (ethanesulfonyl)methylmagnesium chloride after 2 hours, and 89.7% after 4 hours.

### [Example 5]

Samples a and b were obtained in the same manner as in Example 2, with the exception that the temperature in the reactor I after the completion of the dropwise addition was changed from 50°C to 40°C. From the integrated values of the peaks derived from the methyl groups in (methanesulfonyl)ethane by ¹H-NMR for samples a and b, the deuteration percentages were 88.6% D after 2 hours and 99.4% D after 4 hours. In other words, 88.6% of (methanesulfonyl)ethane was converted to (ethanesulfonyl)methylmagnesium chloride after 2 hours, and 99.4% after 4 hours.

### [Example 6]

Samples a and b were obtained in the same manner as in Example 4, with the exception that the tetrahydrofuran solution of methylmagnesium chloride with a concentration of 2.81 M (3.5 mL, 9.8 mmol) was changed to a tetrahydrofuran solution of methylmagnesium chloride with a concentration of 2.81 M (4.2 mL, 11.76 mmol). From the integrated values of the peaks derived from the methyl groups in (methanesulfonyl)ethane by ¹H-NMR for samples a and b, the deuteration percentages were 90.3% D after 2 hours and 98.3% D after 4 hours. In other words, 90.3% of (methanesulfonyl)ethane was converted to (ethanesulfonyl)methylmagnesium chloride after 2 hours, and 98.3% after 4 hours.

As shown in the Examples, an (ethanesulfonyl)methylmagnesium halide can be obtained from (methanesulfonyl)ethane at a high yield by the method for producing an (ethanesulfonyl)methylmagnesium halide according to the present invention.

### INDUSTRIAL APPLICABILITY

A novel method for producing a 2-(ethanesulfonyl)-1-heteroarylethan-1-one compound, which is one of the heterocyclic building blocks used for the organic synthesis of compounds that serve as drug substances for medicines, agricultural chemicals, and the like can be provided.

## Claims

1. A method for producing a 2-(ethanesulfonyl)-1-heteroarylethan-1-one compound, the method comprising chemically reacting an (ethanesulfonyl)methylmagnesium halide with a heteroaryl carboxylic acid ester compound.

2. The method according to Claim 1, further comprising chemically reacting (methanesulfonyl)ethane with an alkylmagnesium halide to obtain said (ethanesulfonyl)methylmagnesium halide.

3. The method according to Claim 1 or 2,
wherein said heteroaryl carboxylic acid ester compound is a substituted or unsubstituted 5- to 6-membered heteroaryl carboxylic acid alkyl ester or a substituted or unsubstituted fused bicyclic 9-membered heteroaryl carboxylic acid alkyl ester.

4. A method for producing an (ethanesulfonyl)methylmagnesium halide, the method comprising chemically reacting (methanesulfonyl)ethane with an alkylmagnesium halide.
